# EUROPEAN PATENT SPECIFICATION

(11) **EP 1 109 538 B1**
(45) Date of publication and mention of the grant of the patent: **02.05.2003**
(21) Application number: 99944682.6
(22) Date of filing: 10.09.1999
(51) Int. Cl.: A61K 9/50, A61K 31/557

(54) **ANTI-INFLAMMATORY PHARMACEUTICAL FORMULATIONS**
ENTZÜNDUNGSHEMMENDE PHARMAZEUTISCHE FORMULIERUNGEN
PREPARATIONS PHARMACEUTIQUES ANTI-INFLAMMATOIRES

(30) Priority: 10.09.1998 GB 9819685
(43) Date of publication of application: 27.06.2001
(73) Proprietor: NORTON HEALTHCARE LIMITED, London E16 2QJ (GB)
(72) Inventor: AUSTIN, John Woolfe, North Weald, Essex CM19 6DL (GB); McINTYRE, Gordon, Bishops Stortford, Herts CM23 3PW (GB); SHETH, Nitin Vadilal, Goshen, NY 10924 (US)
(74) Representative: Wright, Robert Gordon McRae
(86) International application number: GB9902831
(87) International publication number: WO00015200

(56) References cited:
- WO-A-91/16895
- WO-A-99/12524
- US-A- 5 232 704

## Description

This invention relates to pharmaceutical formulations of anti-inflammatory drugs, particularly non-steroidal anti-inflammatory drugs (NSAIDs).

These NSAIDs are used for the treatment of inflammatory conditions such as osteoarthritis or rheumatoid arthritis. A side effect of the oral administration of NSAIDs particularly with long term usage, is a liability to ulcerogenic effects. NSAID induced ulcers in the stomach are potentially dangerous because few or no symptoms may be detected until significant damage has been caused. Certain prostaglandins, for example misoprostol have been shown to reduce and even prevent such ulcers.

Various patent applications relate to use of misoprostol with immediate release drugs, for example GB-A-2135881 (Farmitalia Carlo Erba), WO91/16896 (G D Searle), or where a gastric resistant coating is put over the NSAID in an attempt to reduce further gastric erosion due to release in the stomach of the NSAID, for example WO91/16895, WO91/168 (G D Searle).

There is an increasing use of sustained release preparations of NSAID drugs to reduce the number of doses required by the patient each day. Although the theory of such preparations is that the majority of the drug is released in the intestine rather than the stomach, in practice there is a significant occurrence of gastric problems. This may be due to release of small amounts of drug within the stomach.

The incorporation of misoprostol into such products to reduce the potential for such problems has not previously been disclosed.

According to the present invention there is provided an oral pharmaceutical capsule including a mixture of a delay release formulation of a non-steroidal anti-inflammatory drug (NSAID) and a mixture containing a prostaglandin and one or more excipients, wherein the prostaglandin mixture is a powder, and the capsule contains multi-particulate beads of the NSAID formulation together with the powdered prostaglandin mixture.

The delay release NSAID formulation preferably comprises coated beads or pellets.

The prostaglandin mixture may be provided in the form of a powder which is mixed with the NSAID formulation within the dosage form.

The capsule contains multi-particulate beads of the NSAID formulation together with the powdered prostaglandin mixture. The NSAID beads preferably have coatings adapted to provide programmed release according to the position in the gastrointestinal tract. Use of such coated beads provides a more repeatable release along the gastrointestinal tract and may reduce gastric erosion because the small pellets or beads are easily moved and do not adhere readily to the folds of the gastric wall.

Beads for use in accordance with this invention may have a single slowly erodible coat or may comprise mixtures of beads with differing levels or types of coating adapted to provide a continuous or distributed release profile through the gastrointestinal tract. The delay afforded may range from a minimal delay to several hours, dependent on the pH of the gastrointestinal tract in the immediate vicinity.

The NSAID is preferably but not exclusively one of reasonably low weight per standard dose, that is 200 mg or below. Examples of suitable NSAIDs include tiaprofenic acid, piroxicam, flubiprofen, tenoxicam, meloxicam or similar molecules. Salts or other derivatives of these drugs may be employed in a conventional manner. Most preferably the drug is diclofenac sodium, ketoprofen or indomethacin. Mixtures may be used.

It is possible to produce the pellets or beads by conventional means. Techniques that can be used can include coating the drug on a non-pariel core preferably composed of inert sugar or similar substance and then overcoating with the required coating before encapsulation. The following steps may be employed.
i. Preparation of inert core by conventional pan coating method
ii Active coating by using rotary type fluidized bed.
iii Protective coating by using rotary type fluidized bed.
iv Enteric coating by using rotary type fluidized bed.

The procedure disclosed in EP-A-519144 may be used.

Drug delivery using capsules avoids a further compression step as may be necessary during tablet manufacture.

An alternative method is to form beads or pellets by co-acervation or alternatively by precipitation from solution as described by Zaniboni, Fell and Collett, (Int.J.Pharm, 1995, 125, 151-5).

In a preferred technique the beads may be formed by spheronisation, rotogranulation or a similar technique. If tablets are to be made, preferably the beads should be soft enough to deform slightly under compression to avoid cracking but not too soft so as to deform significantly as deformation may also cause cracking or rupture of the coat. A mixture of drug with a suitable amount of an excipient or excipients can be found by simple experiments. Suitable excipients include polyvinyl pyrolidone, sugars and cellulose derivatives particularly microcrystalline cellulose.

The coating for the beads may include cellulose derivatives eg hydroxypropyl methyl cellulose, methacrylic acid and derivatives eg methyl methacrylates for example, Eudragrit® (Rhom Pharm), especially Eudragrit L or S. Other standard enteric coating materials may be used for example phthalate, eg cellulose acetate phthalate or preferably hydroxypropylacetate phthalate or polyvinylacetate phthalate. Mixtures of these and other materials may be used to produce delay release coated beads. Normally the coating will include plasticisers eg polyethylene glycol, triacilin or phthalate esters.

The prostaglandin component preferably contains misoprostol optionally together with one or more inert excipients. The prostaglandin is normally provided as a 1:10 or 1:100 dilution on an inert cellulose or other binder or filler. Especially useful material for this invention is hydroxypropyl methyl cellulose. The dosage of prostaglandin may be chosen to be suitable to prevent or reduce stomach ulceration caused by the NSAID. A suitable dose of misoprostol is between 10 - 50 µg preferably 50 - 200 µg per dosage form but this may be increased or decreased depending on the NSAID used.

The dosage forms comprise capsules, preferably hard gelatin capsules.

In preferred embodiments of the invention, the potential for gastric erosion is reduced by ensuring that the prostaglandin is released before the NSAID. Any beads for immediate or rapid release are coated with an inert coating which defer solubility in gastric fluid, for example for a period of 30 minutes. Such materials include cellulose derivatives for example hydroxypropyl methyl cellulose, methyl or ethyl celluloses or other se4alants eg Zein. Thin coatings of methacrylate derivatives eg polyhydroxymethacrylate or other materials such as hardened gelatine, waxes, starches or polyvinyl pyrolidone may be used. Other portions of the beads may be coated with methacrylate derivatives, phthalate, for example hydroxypropyl methyl cellulose phthalate or similar materials to give an appropriate release profile as is well known in the art

The invention is further described by means of example, but not in any limitative sense.

### Example 1

Hard gelatin capsules fill were prepared containing a mixture of the following:

| | |
|---|---|
| delay release ketoprofen beads | 250 mg |
| misoprostol (diluted 1:100 on hydroxypropylmethylcellulose) | 20 mg |
| lactose (anhydrous) | 160 mg |
| hydrogenated vegetable oil | 4 mg |

The beads were prepared by spray coating a suspension or solution of ketoprofen onto a non-pareil sugarcore, together with a binder dg polyvinylpyrollidone or hydroxypropylmethyl cellulose. The beads were subsequently coated with a delay release coating eg methylmethacrylate (eg Eudragit (Trade Mark)). Mixtures of beads with various levels of coating were used to give the required therapeutic release pattern.

In a fluidized bed apparatus, uniform spherical inert sugar sphere cores were coated with a first layer consisting of the compounds, an inert water soluble polymer such as hydroxy-propylmethylcellulose or hydroxypropylcellulose, and talc. The second layer consisted of an inert water soluble polymer such as hydroxypropylmethylcellulose or hydroxypropylcellulose, talc and a pigment such as titanium dioxide. The third and enteric coating layer consisted of an enteric coating p9olymer such as co-polymerized methacrylic acid/methacrylic acid methyl esters, a plasticiser such as triethylitrate or similar plasticisers, and talc.

The layers were applied by conventional fluidized bed coating techniques using aqueous solutions or dispersions.

Pseudo zero release was obtained by use of a mixture of beads released at various pHs or at various times dependent on the type of coating.

The beads in Example 1 contained 40% ketoprofen giving a dose per capsule of 100 mg plus 100 µg misoprostol.

The mix was then filled into suitable hard gelatine capsules.

### Example 2

The following formulation was employed:

| | |
|---|---|
| delay release diclofenac beads microcrystalline cellulose (dried) | 214 mg |
| eg Avicel R PH112 | 150 mg |
| misoprostol (1 in 100 dilution on HPMC) | 20 mg |
| stearic acid | 4 mg |
| talc | 8 mg |

Beads containing 35% diclofenac sodium ie 75 mg drug per dose were prepared.

The beads were formed as previously described, or by mixing with a bulking agent eg microcrystalline cellulose, moistening with water, extruding and spheronising to give spherical or ovoid particles about 0.5 mm to 1.5 mm in diameter. These were dried and coated as previously described using a standard coating agent. The beads were mixed as required to give the required release profile.

The beads are usually provided with a coating to prevent immediate release in the stomach, particularly release before the misoprostol has dissolved.

## Claims

1. An oral pharmaceutical capsule from including a mixture of a delay release formulation of a non-steroidal anti-inflammatory drug (NSAID) and a mixture containing a prostaglandin and one or more excipients, wherein the prostaglandin mixture is a powder and the capsule contains multi-particulate beads of the NSAID formulation together with the powdered prostaglandin mixture.

2. A dosage form as claimed in claim 1, wherein the NSAID formulation comprises coated pellets or beads.

3. A dosage form as claimed in claim 1, wherein the prostaglandin is misoprostol.

4. A dosage form as claimed in any preceding claim comprising a mixture of beads with different levels or types of coating.

5. A dosage form as claimed in claim 1, wherein the NSAID is selected from the group consisting of tiaprofenic acid, piroxicam, flubiprofen, tenoxicam, meloxicam and salts and derivatives thereof.

6. A dosage from as claimed in claim 5, wherein the NSAID is selected from the group consisting of diclofenac sodium, ketoprofen and indomethacin and mixtures thereof.

7. A dosage form as claimed in any of claims 3 to 6, wherein the dosage of misoprostol is 50 to 200 µg per dosage form.

8. A dosage form as claimed in any preceding claim, wherein the pellets or beads comprise coatings including the drug on non-pareil cores.

9. A dosage form as claimed in any preceding claim, wherein the pellets or beads are made by co-acervation or precipitation from solution.

10. A dosage form as claimed in claim 8, wherein the beads are made by spheronisation or rotogranulation.

11. A dosage form as claimed in any of claims 8 to 10, wherein the coating includes the drug and an excipient selected from the group consisting of: polyvinyl pyrolidone, sugars and cellulose derivatives.

12. A dosage form as claimed in any preceding claim, wherein the pellets or beads have a coating of compounds selected from the group consisting of: hydroxypropyl methyl cellulose, methacrylic acid and derivatives, methyl methacrylates, cellulose acetate phthalate, hydroxypropylacetate phthalate, polyvinylacetate phthalate and mixtures thereof.

13. A dosage form as claimed in claim 12, wherein the coating includes a plasticiser selected from the group consisting of: polyethylene glycol, triacilin or phthalate esters.

14. A dosage form as claimed in claim 1 comprising a filled hard gelatin capsule.

## Patentansprüche

1. Orale pharmazeutische Kapsel, eine Mischung einer Formulierung mit verzögerter Freisetzung eines nichtsteroidalen entzündungshemmenden Arzneimittels (NSAID) und eine ein Prostaglandin und ein oder mehrere Vehikel enthaltende Mischung einschliessend, worin the Prostaglandinmischung ein Pulver ist und die Kapsel Mehrpartikelperlen der NSAID-Formulierung zusammen mit der pulverförmigen Prostaglandinmischung enthält.

2. Dosierungsform, wie in Anspruch 1 beansprucht, worin die NSAID-Formulierung beschichtete Pellets oder Perlen unfasst.

3. Dosierungsform, wie in Anspruch 1 beansprucht, worin das Prostaglandin Misoprostol ist.

4. Dasierungsform, wie in einem beliebigen vorangehenden Anspruch beansprucht, eine Mischung von Perlen mit verschiedenen Niveaus oder Typen von Beschichtung umfassend.

5. Dosierungsform, wie in Anspruch 1 beansprucht, worin das NSAID aus der Gruppe ausgewählt ist, die aus Tiaprofensäure, Piraxicam, Flubiprofen, Tenoxicam, Meloxicam und deren Salzen und Abkömmlingen besteht.

6. Dosierungsform, wie in Anspruch 5 beansprucht, worin das NSAID aus der Gruppe ausgewählt ist, die aus Diclofenac-Natrium, Ketoprofen und Indometacin und deren Mischungen besteht.

7. Dosierungsform, wie in einem beliebigen der Ansprüche 3 bis 6 beansprucht, worin die Dosierung von Misoprostol 50 bis 200 µg pro Dosierungsform beträgt.

8. Dosierungsform, wie in einem beliebigen vorangehenden Anspruch beansprucht, worin die Pellets oder Perlen Beschichtungen umfassen, die das Arzneimittel auf Nonpareille-Kernen einschliessen.

9. Dosierungsform, wie in einem beliebigen vorangehenden Anspruch beansprucht, worin die Pellets oder Perlen durch Koazervation oder Fällung aus einer Lösung hergestellt werden.

10. Dosierungsform, wie in Anspruch 8 beansprucht, worin die Perlen durch Sphäronisierung oder Rotogranulierung hergestellt werden.

11. Dosierungsform, wie in einem beliebigen der Ansprüche 8 bis 10 beansprucht, worin die Beschichtung das Arzneimittel sowie ein Vehikel enthält, das aus der Gruppe ausgewählt ist, die aus Polyvinylpyrrolidon, Zuckern und Celluloseabkömmlingen besteht.

12. Dosierungsform, wie in einem beliebigen vorangehenden Anspruch beansprucht, worin die Pellets oder Perlen eine Beschichtung von Verbindungen besitzen, die aus der Gruppe ausgewählt sind, die aus Hydroxypropylmethylcellulose, Methacrylsäure und Abkömmlingen, Methylmethacrylaten, Celluloseacetatphthalat, Hydroxypropylacetatphthalat, Polyvinylacetatpathalat und deren Mischungen besteht.

13. Dosierungsform, wie in Anspruch 12 beansprucht, worin die Beschichtung einen Weichmacher einschliesst, der aus der Gruppe ausgewählt ist, die aus Polyethylenglykol, Triacilin oder Phthalatestern besteht.

14. Dosierungsform, wie in Anspruch 1 beansprucht, eine gefüllte harte Gelatinekapsel umfassend.

## Revendications

1. Capsule pharmaceutique orale comprenant un mélange d'une formulation retard d'un médicament anti-inflammatoire non-stréroïdien (MAINS) et un mélange contenant une prostaglandine et un ou plusieurs excipients, dans laquelle le mélange de prostaglandine est en poudre et la capsule contient des billes multi-particules de la formulation MAINS avec le mélange pulvérulent de prostaglandine.

2. Forme de dosage selon la revendication 1, dans laquelle la formulation MAINS comprend des pillules or des billes revêtues.

3. Forme de dosage selon la revendication 2, dans laquelle la prostaglandine est le misoprostol.

4. Forme de dosage selon l'une quelconque des revendications précédentes, comprenant un mélande de billes ayant des niveaux ou des types de revêtements différents.

5. Forme de dosage selon la revendication 1, dans laquelle le MAINS est choisi dans le groupe consistant en l'acide tiaprofénique, le flubiprofène, le toxicam, le meloxicam et leurs sels et dérivés.

6. Forme de dosage selon la revendication 5, dans laquelle le MAINS est choisi dans le groupe consistant en le diclofenac sodium, le cétoprofène et l'indométhacine, et leurs mélanges.

7. Forme de dosage selon l'une quelconque des revendications 3 à 6, dans laquelle le dosage du misoprostol est de 50 à 200 µg par forme de dosage.

8. Forme de dosage selon l'une quleconque des revendications précédentes, dans laquelle les pillules ou billes comprennent des revêtements contenant le médicament sur les noyaux non-pareils.

9. Forme de dosage selon l'une quelconque des revendications précédentes, dans laquelle les pillules ou billes sont fabriquées par co-acervation or précipitation à partir d'une solution.

10. Forme de dosage selon la revendication 8, dans laquelle les billes sont fabriquées par sphéronisatlon ou rotogranulation.

11. Forme de dosage selon l'une quelconque des revendications 8 à 10, dans laquelle le revêtement comprend le médicament et un excipient choisi dans le groupe consistant en la polyvinylpyrolidone, les sucres et les dérivés de cellulose.

12. Forme de dosage selon l'une quleconque des revendications précédentes, dans laquelle les pillules ou billes comprennnent un revêtement de composées choisis dans le groupe consistant en l'hydroxypropylméthyl cellulose, l'acide méthacrylique et ses dérivés, les méthacrylates de méthyle, l'acétate phtalate de cellulose, l'acétate phtalate d'hydroxypropyle, l'acétate phtalate de polyvinyle et leurs mélanges.

13. Forme de dosage selon la revendication 12, dans laquelle le revêtement comprend un plastifiant choisi dans le groupe consistant en le polyéthylène glycol, la triaciline ou les esters phatlates.

14. Forme de dosage selon la revendication 1, comprenant une capsule remplie de gélatine dure.
